# EUROPEAN PATENT SPECIFICATION

(11) **EP 2 701 522 B1**
(45) Date of publication and mention of the grant of the patent: **02.10.2019**
(21) Application number: 11724295.8
(22) Date of filing: 29.04.2011
(51) Int. Cl.: A23C 9/123, C12R 1/225

(54) **USE OF NISIN RESISTANT MUTANT STRAINS OF LACTOBACILLI FOR REDUCING THE POST ACIDIFICATION IN FOOD PRODUCTS**
VERWENDUNG VON NISIN-RESISTENTEN MUTANTEN LACTOBACILLUS-STÄMMEN ZUR REDUZIERUNG DER NACHSÄUERUNG BEI NAHRUNGSMITTELN
UTILISATION DE SOUCHES MUTANTES RÉSISTANTES À LA NISINE DE LACTOBACILLES POUR RÉDUIRE LA POST-ACIDIFICATION DANS DES PRODUITS ALIMENTAIRES

(43) Date of publication of application: 05.03.2014
(73) Proprietor: Compagnie Gervais Danone, 75009 Paris (FR)
(72) Inventor: DRUESNE, Anne, F-91190 Villiers-le-Bacle (FR); GARAULT, Peggy, F-91130 Montlhery (FR); FAURIE, Jean-Michel, F-78350 Jouy-en-Josas (FR)
(74) Representative: Gevers & Orès
(86) International application number: PCT/IB2011/051902
(87) International publication number: WO 2012/146953

(56) References cited:
- EP-A1- 1 273 237
- EP-A1- 2 294 926
- EP-A2- 0 712 935
- US-A- 5 059 431
- US-A- 5 683 890
- TAKALA T. ET AL: "A food-grade cloning vector for lactic acid bacteria based on the nisin immunity gene nisI", APPLIED MICROBIOLOGY AND BIOTECHNOLOGY, vol. 59, no. 4-5, December 2002 (2002-12), pages 467-471, XP055018982, ISSN: 0175-7598, DOI: 10.1007/s00253-002-1034-4
- BOSSI M G ET AL: "Effect of nisin on lactic acid bacteria detected by conductance measurement. (translated)", SCIENZA E TECNICA LATTIERO-CASEARIA, vol. 39, no. 4, 1988, pages 249-261, XP002669224, ISTITUTO SPERIMENTALE LATTIERO-CASEARIO, LODI, MILAN, ITALY
- DELVES-BROUGHTON J ET AL: "Applications of the bacteriocin, nisin", ANTONIE VAN LEEUWENHOEK, vol. 69, no. 2, 1 January 1996 (1996-01-01), pages 193-202, XP009126082, SPRINGER NETHERLANDS, DORDRECHT ISSN: 0003-6072, DOI: 10.1007/BF00399424
- DATABASE FSTA [Online] INTERNATIONAL FOOD INFORMATION SERVICE (IFIS), FRANkFURT-MAIN, DE; SENESI E ET AL: "Nisinogenic streptococci and their use in the making of Provolone cheese.", XP002669228, Database accession no. FS-1975-08-P-1827
- SENESI E ET AL: "STREPTOCOCCHI NISINOGENI E LORO IMPIEGO NELLA TECNOLOGIA DEL FORMAGGIO PROVOLONE", IL LATTE, TECNICHE NUOVE, MILAN, IT, 1 February 1975 (1975-02-01), pages 84-86, XP001058163, ISSN: 0392-6060

## Description

The present invention relates to the reduction of the post-acidification in food products.

During the preservation, in particular storage and transportation of food products containing live microorganisms, the microbial metabolic activity is mainly expressed via acid production which leads to quality deterioration of product, in particular over-acidic taste at the end of the shelf life. This mechanism of acidification is called post-acidification and is well known. This is especially a problem in products where no extra sugars or flavours have been added.

To reduce this phenomenon, products have to be stored at low temperatures, for example at 10°C. In emerging countries, it is not always possible to store at a low temperature and to insure a complete cold chain and this has the disadvantage that the shelf life of food products is drastically reduced.

Also, the prevention of the post-acidification is a crucial issue for food industries.

Up to now, a number of efforts have been reported to reduce acid production by reducing the number of the active cells in starter culture or to eliminate living cells from the finished product by pasteurizing. In some of these studies, it has been shown that an antibacterial agent nisin secreted by *Lactococcus lactis subsp. lactis* in a medium suppresses the growth of the starter bacteria and control formation of acids. In EP0505164, fermented milk can be manufactured by the addition to raw milk of nisin-producing lactic acid bacteria belonging to genus *Lactococcus lactis* together with other lactic acid bacteria to be used for the fermentation of the raw milk. Nisin produced and accumulated in the fermented milk suppresses the growth of bacteria which cause acid formation, thus controlling the acidity increase during storage and transportation. Kalra et al. (Indian Journal of Dairy Science 28: 71-72 (1975)) incorporated the nisin producing culture *Streptococcus lactis* (now known as *L. lactis subsp. lactis*) along with the yogurt culture before fermentation. EP2294926 discloses the addition of bacteriocin-producing lactic acid bacteria to a yoghurt mix which are then killed before fermentation. Others introduced nisin in milk prior to fermentation (Bayoumi, Chem. mikrobiol. technol. lebensm. 13:65-69 (1991)) or following fermentation (Gupta et al., Cultured Dairy Products Journal 23: 17-18 (1988); Gupta et al., Cultured Dairy Products Journal 23: 9-10 (1989)). In all cases, the rate of post- acidification was only partially inhibited by these treatments and the fermented milk continued to become more acidic throughout its shelf life. Attempts to arrest the production of acid by yogurt cultures, by the addition of nisin or a nisin producing culture before or after milk fermentation have not been successful. As mentioned above, this use affects the living bacteria which have a great interest for the food industry. In particular, strains of the *Lactobacillus casei* group have been reported to have health-promoting properties, and are used as probiotics in different food products. Moreover, the addition of nisin producing culture (*L. lactis*) is not possible in case of yoghurt production in order to conserve the denomination "yoghurt".

Secondly, several low acidification strains have been described in prior art to control acidification and post-acidification of fresh fermented product.

Post-acidification results essentially from the use, by the bacteria, of the lactose remaining in the product. In order to prevent it, it has been proposed to use strains of lactic acid bacteria which do not ferment lactose, or ferment it very little. Patents EP 1078074 and EP 1893032] discloses lactic acid bacteria which are no longer able to ferment lactose or with low fermentation capacities during storage.. Respectively, patent EP 1078074 relates to *L. bulgaricus* mutants deficient in beta-galactosidase activity, comprising nonsense mutation in at least one of the genes of the lactose operon and the patent EP 1893032 concerns mutants modified in their lactose transport activity by mutation on the lactose permease. However, these solutions cannot be used in fermented vegetable or fruit products, or in dairy products comprising other sugars than lactose.

EP1802652 discloses the solution to decrease the population of lactic acid bacteria at the end of fermentation process by manipulating the concentration of amino acids. But this solution has a cost in term of amino acids or specific peptides mandatory added to display a good growth of lactic acid bacteria. This solution is demonstrated only for *Streptococcus thermophilus* which limit applications.

In JP7236416 a solution was developed using neomycin resistant mutants of lactic acid bacteria having an altered H⁺-ATPase activity. Bacteria are supposed to stop acidification when they are no more able to generate a pH gradient between the medium and the cellular cytoplasm. However, the use of antibiotic resistance bacteria is always questionable for a safety point of view. Usually, antibiotic resistance is a criterion for strain rejection during a strain selection process.

All solutions present advantages and also drawbacks depending on the type of applications. Hence, new solutions to avoid excessive post acidification are welcome in order to have different tools for different species for reducing post-acidification.

The inventors surprisingly found that when nisin resistant mutants from lactic acid bacteria, in particular from *L. helveticus* and *L. bulgaricus,* are added in food products, the post acidification is reduced.

An objet of the present invention is the use of at least one nisin resistant mutant in food products for reducing post acidification.

Another aim of the invention is to provide a method for reducing post acidification in food products and a food product obtainable by this method.

The present invention relates to the use of at least one strain of living nisin-resistant lactobacilli for reducing post-acidification in food product.

The term "lactobacilli" designates all the species of the genus *Lactobacillus subsp.* They represent an important part of the lactic acid bacteria group at the industrial level and are particularly used in the fermentation of dairy products in combination with *Streptococcus thermophilus.* They are also used as probiotic in several food products in order to give health-promoting properties (Lebeer *et al.,* 2008) [ref: Lebeer S., Vanderleyden J., and De Keersmaecker S. C. J. Genes and molecules of lactobacilli supporting probiotic action. Microbiology And Molecular Biology Reviews, Dec. 2008, 72(4), p. 728-764].

The lactobacilli are typically selected from the group consisting of *Lactobacillus helveticus, L. delbrueckii subsp. bulgaricus, L. delbrueckii subsp. delbrueckii, L. delbrueckii subsp. lactis, L. casei, L. paracasei, L. acidophilus, L. rhamnosus, L. plantarum, L. reuteri, L. brevis,* and *L. fermentum.*

Preferably, it belongs to the species *L. helveticus, L.delbrueckii and their* subspecies, in particular *L. delbrueckii subsp. bulgaricus.*

Particular preferred lactobacilli are chosen from the group consisting of CNCM 1-4452, CNCM 1-4453 and CNCM 1-4454.

The lactobacilli used in the invention are preferably obtained from the mother strains chosen from the group consisting of *L. bulgaricus* CNCM 1-2836 and *L. helveticus* CNCM 1-3435.

In the present invention, the lactobacilli used are living bacteria. The term "living" in the invention designates lactobacilli which are alive at the time of use and in the product until the end of the shelf live of the product, in particular until 28 days at a temperature of 10°C. This state is important, in particular in yogurts for which the official guidelines (the codex Alimentarius (prepared by the codex Alimentarius Commission under the aegis of the FAO and ODM content, and published by the Information Division of the FAO, available on-line at http://www.codexalimentarius.net require that a product will be designated a yogurt if it contain at least one strain of S. *thermophilus* and at least one strain of *L. bulgaricus* in the live form in an amount of at least 1 ^{∗} 10⁷ cfu/g of the lactic portion.

Strains of lactobacilli which are more particularly suitable for use in the present invention have a nisin resistance of at least 6.25 µg/ml M.I.C in particular at least 12.5 µg/ml M.I.C. The term "MIC" defines the minimum inhibitory concentration and designates herein, the lowest concentration of nisin which inhibits the growth of lactobacilli after overnight incubation. The MIC can be determined by several methods well known like microdilution method in liquid or solid medium (Klare et al., 2005). *Ref:* Ingo Klare, Carola Konstabel, Sibylle Müller-Bertling, Rolf Reissbrodt, Geert Huys, Marc Vancanneyt, Jean Swings, Herman Goossens, and Wolfgang Witte Evaluation of New Broth Media for Microdilution Antibiotic Susceptibility Testing of Lactobacilli, Pediococci, Lactococci, and Bifidobacteria Applied and Eenvironmental Microbiology, 2005, 8982-8986, 71(12*).*

As it is well known in the art, nisin is a food grade anti-microbial peptide, a lantibiotic, produced by some strains of *Lactococcus lactis* and is active against a broad range of Gram-positive bacteria. Nisin has gained importance in the food industry, where it is used to control the growth of spore formers in preserves and of unwanted microbial flora in dairy products.

In the invention, the nisin is not used as mentioned above, but is used as selective agent for selecting strains with low post-acidification properties. The term "nisin resistant lactobacilli" as used herein, designates strains selected for growth in the presence of nisin. In other words, strains which are capable of growing with nisin have been isolated from a culture of wild type lactobacilli.

In the present invention, the term "food product" designates products chosen from the group consisting dairy products, fruit juices, vegetable products, infant formulas, milk powders.

According to a particular embodiment the food product is fermented. A "fermented food product" is a product having undergone at least a step of fermentation.

According to a particular embodiment the fermented products are chosen from the group consisting of fermented dairy products, fermented juices, fermented vegetable products.

The term "fermented juices" refers to product obtained by fermentation from fruit juices, in particular orange juice, apple juice, lemon juice, pear juice.

The term "fermented vegetable products" refers to product obtained by fermentation from vegetable juices including soya juice, oat juice and rice juice, or by fermentation of vegetal milk source or solid state fermentation of vegetables.

According to a more particular embodiment the fermented dairy products are chosen from the group consisting of yoghurts, fermented milks, fermented infant milks, fermented drinks. The term "fermented milks" and "yogurts" have the usual meanings attributed to them in the dairy industry, i.e. products which are intended for animal consumption, more particularly human consumption, and which are derived from acidifying lactic fermentation of a dairy substrate (animal milk, in particular cow milk). Said products may contain secondary ingredients such as fruits, vegetables, sugars, flavors, starch, thickeners, etc, provided that these ingredients are suitable for human or animal consumption. More particularly, the denomination "fermented milk" (decree n.deg 88.-1203 of December 30th, 1988) is reserved for a dairy product prepared with skimmed milks or not, or condensed milks or powders some, having undergone a heat treatment at least equivalent to pasteurization, and sown with producing micro-organisms of lactic acid such as the lactobacilli (*Lactobacillus acidophilus, L. casei, L, plantarum, L. reuteri, L. johnsonii*), the certain streptococci (*Streptococcus thermophilus*) bifidobacteria (*Bifidobacterium bifidum, B. longum, B. short, B. animalis*) and the lactococci ones. Moreover, the term "yogurt" (yoghourt) is reserved for the fermented milk obtained, using standard methods, by the development of specific thermophilic lactic bacteria designated *Lactobacillus bulgaricus* (also designated *Lactobacillus delbrueckii subsp. bulgaricus*) and *Streptococcus thermophilus,* which must be alive in the finished product, in an amount of at least 1.10⁷ cfu of *S. thermophilus* and *L. bulgaricus* bacteria per gram of product, expressed as the lactic portion of the product.

Preferably, according to the invention, the fermented food products are fermented with at least one strain of nisin resistant lactobacilli.

According to a more particular embodiment the food product comprises other living bacteria chosen from the group consisting of *Streptococcus spp.*; *Lactobacillus spp,* in particular *L. bulgaricus, L. acidophilus* and *L. casei; L. helveticus, Lactococcus spp.* and *Bifidobacterium spp.*

According to a particular embodiment, the food product is stored at room temperature, or at temperatures from 0°C to 25°C, more particularly at temperatures from 4°C to 10°C, more specifically at temperatures from 6°C to 8°C during a period of at least 20 days, in particular of at least 28 days. The food product can be stored alternately at a temperature between 0°C and 25°C and at room temperature or inversely. In the last case, the cold chain is disrupted.

According to the invention, the food product has a reduce post acidification in comparison to food product not using nisin resistant lactobacilli. The expression "reducing post acidification" designates the capacity of nisin resistant lactobacilli to produce less acid during storage of the food product. In the present invention, the nisin resistant lactobacilli have a lower post-acidification than the mother strain from which it is derived. Under the same storage conditions (28 days of storage at 4°C), the ΔpH (difference between the pH at day 0 (D0) and the pH at day 28 (D28)) is of the order of 0,3 in the case of mother strains, and the ΔpH is of the order of 0,1 to 0,2 in the case of mutant strains.

According to the invention, a post-acidification reduction is a reduction of the ΔpH of at least 0,1 pH unity (upH), in particular 0,2 upH .

The present invention also relates to a method for reducing post-acidification in food products wherein said method comprises a step of adding at least one strain of living nisin resistant lactobacilli in a food matrix.

The expression "food matrix" designates the food product before the addition of nisin resistant lactobacilli, in particular designates products chosen from the group consisting dairy products, fruit juices, vegetable products, infant formulas, milk powders.

In an advantageous embodiment, in the method of the invention the lactobacilli are selected from the group consisting of *Lactobacillus helveticus,* , *L. casei, L. paracasei, L. acidophilus, L. rhamnosus, L. plantarum, L. reuteri, L. delbrueckii subsp bulgaricus, L. delbrueckii subsp lactis, L. delbrueckii subsp delbrueckii, L. brevis* and *L. fermentum.*

In a more advantageous embodiment, in the method of the invention the lactobacilli are selected from the group consisting of CNCM 1-4452, CNCM 1-4453 and CNCM 1-4454.

In a particular embodiment, in the method of the invention the strain has a nisin resistance of at least 6.25 µg/ml M.I.C, in particular at least 12.5 µg/ml M.I.C.

According to preferred embodiment, in the method of the invention, the addition of the nisin resistant lactobacilli is done during, before or after the step of fermentation of the fermented food product. In the case of unfermented product, the addition of the nisin resistant lactobacilli is done before storage.

According to a particular embodiment the fermented products are chosen from the group consisting of fermented dairy products, fermented juices, and fermented vegetable products.

According to a more preferred embodiment, in the method of the invention, the fermentation is carried out in the presence of at least one other living bacterium.

The said living bacteria are chosen from the group consisting of *Streptococcus spp.*; *Lactobacillus spp,* in particular *L. bulgaricus, L. acidophilus* and *L. casei; L. helveticus, Lactococcus spp.* and *Bifidobacterium spp.*

According to a preferred embodiment, the method of the invention comprises the following steps:
a. Providing a strain of living nisin resistant lactobacilli,
b. Inoculating a food matrix with the strain obtained in step a),
c. Fermenting the inoculated food at a temperature of from 4°C to 50°C, until it reaches a desired target pH
d. Recovering and storing the product obtained in step c), at a temperature of from 4 to 40°C during at least 28 days.

The pH target may have a value between 3, 6 to 5.0.

It is also disclosed a food product obtainable by the process of the invention.

The product of the invention is preferably a fermented dairy product.

The present invention also relates to a mutant strain of *L. bulgaricus* deposited according to the Treaty of Budapest on March 10th 2011 with the CNCM (Collection Nationale de Culture de Microorganismes [National Collection of Microorganism Cultures] held by the Pasteur Institute under the number 1-4452.

The invention also concerns a mutant strain of *L. bulgaricus,* filed on March 3^{rd} 2011 with the CNCM under the number 1-4453 and a mutant strain of *L. helveticus,* filed on March 10^{th} 2011 with the CNCM under the number 1-4454. Such strains are derived from the *L. bulgaricus* CNCM 1-2836 (deposited with the CNCM on April 4^{th} 2002) and *L. helveticus* CNCM 1-3435 (deposited with the CNCM on May 5th 2005) respectively. The mutant strains CNCM 1-4452, 4453 and 4454 have a nisin resistance of at least 6,25 µg/ml M.I.C, in particular at least 12,5 µg/ml M.I.C.

The present invention also relates to mutant strains of *L. bulgaricus* and *L. helveticus,* characterised in that the said mutants have a nisin resistance of at least 6.25 µg/ml M.I.C, in particular at least 12.5 µg/ml M.I.C.

The present invention therefore relates a lactic ferment (starter) comprising at least one mutant strain as described above. According to a specific embodiment, a lactic ferment according to the invention comprises at least one mutant strain of *L. bulgaricus* and/or *L. helveticus* combined with at least one other lactic acid bacteria strain, for example *Streptococcus spp.; Lactobacillus spp,* in particular *L. bulgaricus, L. acidophilus* and *L. casei; L. helveticus, Lactococcus spp.* and *Bifidobacterium spp.*

### FIGURES

This invention is illustrated by the following figures:

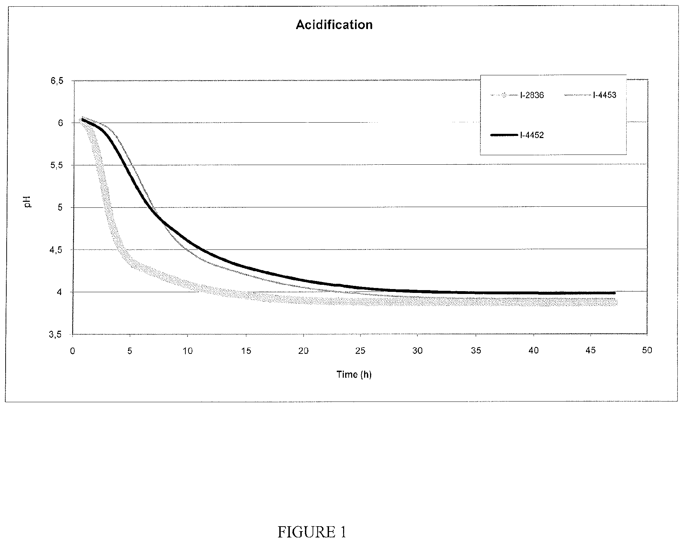

Figure 1: Figure 1 represents the kinetics of acidification of the strains CNCM 1-2836 and clones 1-4452 and 1-4453. The ordinate corresponds to the pH and the abscissa corresponds to the time (in hours). The line in black corresponds to the strains *L. bulgaricus* CNCM 1-4452. The line in gray corresponds to the strains *L. bulgaricus* CNCM 1-4453. The line with circle gray (which gives a tick line gray due to the large number or experimental points) corresponds to the strains *L. bulgaricus* CNCM 1-2836.

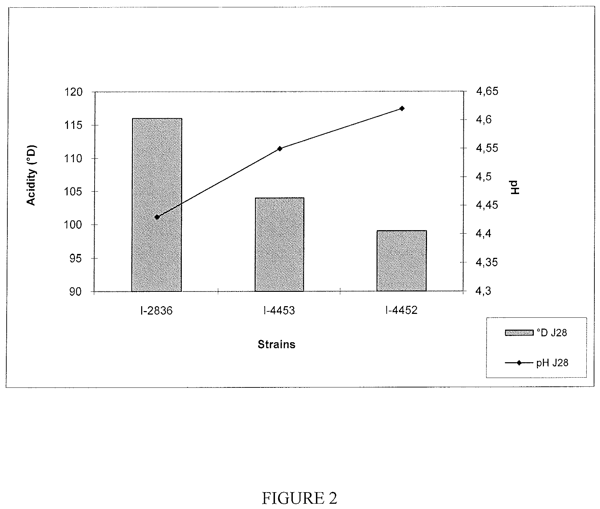

Figure 2: Figure 2 represents the measure of pH and acidity of 3 fresh dairy products (with 1-2836, 1-4453 or 1-4452) after 28 days of storage. The histogram represents the acidity measured at 28 days (in °D) and the curve represents the pH measured at 28 days.

### EXAMPLES

The present invention will be explained with reference to examples which are given for illustration only and are not intended for limiting the invention.

### EXAMPLE 1: OBTAINING NISIN RESISTANT LACTOBACILLI

Mother strains, 1-2836 and 1-3435, chosen in that application are industrial strains used for fermented milk manufacturing showing significant post-acidification during storage.

### 1) Evaluation of the sensitivity of a Lactobacillus bulgaricus and L. helveticus to nisin

To assess the nisin sensibility of the strain *L. bulgaricus* 1-2836, and *L. helveticus* 1-3435 the strains are grown in a rank of MRS tubes containing increasing amounts of nisin. After 16h at 37°C, the optical density of each culture is measured.

### 2) Obtaining nisin resistant lactobacilli

Strains *L. bulgaricus* 1-2836, and *L. helveticus* 1-3435 were grown in MRS medium with an inhibitory concentration of nisine. After 16h at 37°C, 10ml of culture were centrifuged. Pellets were suspended in 100µl of peptone-saline solution and sprayed on agar MRS medium in petri dishes. A piece of blotting paper was placed in the middle of the plate and then soaked with 15µl of a nisine solution 2,5mg/ml. After 72h incubation at 37°C, resistant clones appeared in the inhibition zone around the disc of blotting paper. The clones which were the closest to the disk were recovered for analysis. Notably, their sensibility to nisin was tested in liquid medium using increasing amounts of nisin concentration.

All the clones obtained in particular 1-4452 and 1-4453 and 1-4454 have a minimum inhibitory concentration of at least 6,25 µg/ml µM. This MIC confirms resistance of clones to nisin.

The clones 1-4452 and 1-4453 obtained were then tested by fermentation in milk. The kinetics of acidification provided information about the milk-acidifying capacity of these clones.

### 3) Acidification kinetics in milk with clones derived from strain 1-2836

### a) Fresh dairy product preparation

The starter culture inoculum consists of:
- Strain 1-4452
- Strain 1-4453
- Strain 1-2836

Strains were grown twice in MRS medium containing 6.25µg nisin/ml.

Starter were prepared in 100ml sterilized milk (135g/L milk powder and 2 g/L yeast extract). The inoculation rate was 2% and milks were incubated at 44°C till a Dornic acidity of 70 was reached. The fermented milks were stored one night at 4°C.

Dairy product were prepared from 100.ml sterilized milk, inoculate with the above mixed culture. The cultures were grown at 44°C until they reach a pH value of about 4.75. They were then cooled to 4°C and stored at 4°C during 28 days.

### b) Follow up of acidification kinetics

The acidification kinetic was followed continuously with a CINAC system (Ysebaert, France) which allows continuous measurement of pH values.

It can be seen from FIG 1 that the acidification kinetics are slower for clones than for mother strain 1-2836;

### 4) Measure of pH and acidity of fresh dairy product after 28 days of storage

The measure was done on 3 fresh dairy products containing 2 clones derived from strain 1-2836 (1-4452 and 1-4453) and one containing the strain 1-2836.

There acidity was measured after 28 days of storage at 4°C.

| **Strain** | **Fermentation time** | **pH at the end of the fermentation** | **Acidity (°D) day 0** | **pH day 28** | **Acidity (°D) day 28** |
|---|---|---|---|---|---|
| **I-2836** | 3h25 | 4,74 | 77°D | 4,43 | 116°D |
| **I-4453** | 8h | 4,75 | 75°D | 4,55 | 104°D |
| **I-4452** | 8h25 | 4,75 | 76°D | 4,62 | 99°D |

These results are shown in Figure 2.

The products with nisin resistant lactobacilli have showed only weak post acidification after 28 days.

## Claims

1. Use of at least one strain of living nisin-resistant lactobacilli for reducing post-acidification in food product.

2. The use according to claim 1 wherein the lactobacilli are selected from the group consisting of *Lactobacillus helveticus, L. delbrueckii subsp. bulgaricus, L. delbrueckii subsp. delbrueckii, L. delbrueckii subsp. lactis, L. casei, L. paracasei, L. acidophilus, L. rhamnosus, L. plantarum, L. reuteri, L. brevis,* and *L. fermentum*

3. The use according to claim 2 wherein the lactobacilli are particularly chosen from *L. helveticus, L.delbrueckii* and their subspecies, in particular *L. delbrueckii subsp. bulgaricus.*

4. The use according to claim 3 wherein the lactobacilli are chosen from the group consisting of CNCM 1-4452, CNCM 1-4453 and CNCM I-4454.

5. The use according to claims 1 to 4 wherein the strain has a nisin resistance of at least 6,25 µg/ml Minimum Inhibitory Concentration, in particular at least 12,5 µg/ml Minimum Inhibitory Concentration.

6. The use according to claims 1 to 5 wherein food product is chosen from the group consisting dairy products, milk, fruit juices, vegetable products, infant formulas, milk powder.

7. The use according to claims 1 to 6 wherein fermented products are chosen from the group consisting of fermented dairy products, fermented juices, fermented vegetable products.

8. A method for reducing post-acidification in food products wherein said method comprises a step of adding at least one strain of living nisin resistant lactobacilli in a food matrix.

9. Method according to claim 8 wherein the lactobacilli are selected from the group consisting of *Lactobacillus. helveticus, L. bulgaricus, L. casei, L. paracasei, L. acidophilus, L. rhamnosus, L. plantarum, L. reuteri, L. delbrueckii subsp.. bulgaricus* and *L. fermentum.*

10. Method according to claim 9 wherein the lactobacilli are selected from the group consisting of CNCM 1-4452, CNCM 1-4453 and CNCM I-4454.

11. Method according to claims 8 to 10 wherein the strain has a nisin resistance of at least 6.25 µg/ml Minimum Inhibitory Concentration, in particular at least 12,5 µg/ml Minimum Inhibitory Concentration.

12. Method according to claims 8 to 11 wherein the addition of the nisin resistant lactobacilli is done during, before or after the step of fermentation of the food product.

13. Method according to claims 8 to 12 wherein fermentation is carried out in the presence of at least one other living bacterium.

14. Method according to claims 8 to 13 wherein said other living bacteria is a live lactic bacteria, chosen from the group consisting of *Streptococcus spp.*; *Lactobacillus spp,* in particular *L. bulgaricus, L. acidophilus* and *L. casei; L. helveticus, Lactococcus spp.* and *Bifidobacterium spp.*

15. Method according to anyone of the preceding claims wherein said method comprises:
a. Providing a strain of living nisin resistant lactobacilli,
b. Inoculating a food matrix with the strain obtained in step a),
c. Fermenting the inoculated medium at a temperature of from 4°C to 50°C, until it reaches a desired target pH
d. Recovering and storing the product obtained in step c), at a temperature of from 4°C to 40°C during at least 28 days.

16. Mutant strain of *L. bulgaricus,* filed on March 10^{th} 2011 with the CNCM under the number 1-4452.

17. Mutant strain of *L. bulgaricus,* filed on March 10^{th} 2011 with the CNCM under the number 1-4453.

18. Mutant strain of *L. helveticus,* filed on March 10^{th} 2011 with the CNCM under the number 1-4454.

## Patentansprüche

1. Verwendung von mindestens einem Stamm lebender, nisin-resistenter Lactobazillen zur Reduzierung von Nachsäuerung in Nahrungsmittelprodukt.

2. Verwendung nach Anspruch 1, wobei die Lactobazillen ausgewählt sind aus der Gruppe bestehend aus *Lactobacillus helveticus, L. delbrueckii subsp. bulgaricus, L. delbrueckii subsp. delbrueckii, L. delbrueckii subsp. lactis, L. casei, L. paracasei, L. acidophilus, L. rhamnosus, L. plantarum, L. reuteri, L. brevis,* und *L. fermentum*

3. Verwendung nach Anspruch 2, wobei die Lactobazillen insbesondere gewählt sind aus *L. helveticus, L. delbrueckii* und deren Subspezien, im Besonderen *L. delbrueckii subsp. bulgaricus.*

4. Verwendung nach Anspruch 3, wobei die Lactobazillen gewählt sind aus der Gruppe bestehend aus CNCM I-4452, CNCM I-4453 und CNCM I-4454.

5. Verwendung nach Ansprüchen 1 bis 4, wobei der Stamm eine Nisin-Resistenz von mindestens 6,25 µg/ml minimale Hemmkonzentration, im Besonderen mindestens 12,5µg/ml minimale Hemmkonzentration aufweist.

6. Verwendung nach Ansprüchen 1 bis 5, wobei Nahrungsmittelprodukt gewählt ist aus der Gruppe bestehend Molkereiprodukten, Milch, Fruchtsäften, Gemüseprodukten, Säuglingsanfangsnahrung, Milchpulver.

7. Verwendung nach Ansprüchen 1 bis 6, wobei fermentierte Produkte gewählt sind aus der Gruppe bestehend aus fermentierten Molkereiprodukten, fermentierten Säften, fermentierten Gemüseprodukten.

8. Verfahren zum Reduzieren von Nachsäuerung in Nahrungsmittelprodukten, wobei das Verfahren einen Schritt des Zugebens von mindestens einem Stamm lebender, nisin-resistenter Lactobazillen in eine Nahrungsmittelgrundmasse umfasst.

9. Verfahren nach Anspruch 8, wobei die Lactobazillen ausgewählt sind aus der Gruppe bestehend aus *Lactobacillus helveticus, L. bulgaricus, L. casei, L. paracasei, L. acidophilus, L. rhamnosus, L. plantarum, L. reuteri, L. delbrueckii subsp. bulgaricus,* und *L. fermentum.*

10. Verfahren nach Anspruch 9, wobei die Lactobazillen ausgewählt sind aus der Gruppe bestehend aus CNCM I-4452, CNCM I-4453 und CNCM I-4454.

11. Verfahren nach Ansprüchen 8 bis 10, wobei der Stamm eine Nisin-Resistenz von mindestens 6,25 µg/ml minimale Hemmkonzentration, im Besonderen mindestens 12,5µg/ml minimale Hemmkonzentration aufweist.

12. Verfahren nach Ansprüchen 8 bis 11, wobei das Zugeben der nisin-resistenten Lactobazillen während, vor oder nach dem Schritt des Fermentierens des Nahrungsmittelprodukts erfolgt.

13. Verfahren nach Ansprüchen 8 bis 12, wobei Fermentieren in der Gegenwart von mindestens einem weiteren lebenden Bakterium ausgeführt wird.

14. Verfahren nach Ansprüchen 8 bis 13, wobei das andere lebende Bakterien ein lebendes Milchsäurebakterien ist, gewählt aus der Gruppe bestehend aus *Streptococcus spp.*; *Lactobacillus spp,* im Besonderen *L. bulgaricus, L. acidophilus* und *L. casei; L. helveticus, Lactococcus spp.* und *Bifidobacterium spp.*

15. Verfahren nach einem der vorstehenden Ansprüche, wobei das Verfahren umfasst:
a. Bereitstellen eines Stamms lebender, nisin-resistenter Lactobazillen,
b. Impfen einer Nahrungsmittelgrundmasse mit dem in Schritt a) erhaltenen Stamm,
c. Fermentieren des geimpften Mediums bei einer Temperatur von 4 °C bis 50 °C, bis es einen gewünschten Ziel-pH-Wert erreicht,
d. Gewinnen und Lagern des in Schritt c) erhaltenen Produkts bei einer Temperatur von 4 °C bis 40 °C während mindestens 28 Tagen.

16. Mutanter Stamm von *L. bulgaricus,* der am 10. März 2011 bei der CNCM unter der Nummer I-4452 hinterlegt wurde.

17. Mutanter Stamm von *L. bulgaricus,* der am 10. März 2011 bei der CNCM unter der Nummer I-4453 hinterlegt wurde.

18. Mutanter Stamm von *L. helveticus,* der am 10. März 2011 bei der CNCM unter der Nummer I-4454 hinterlegt wurde.

## Revendications

1. Utilisation d'au moins une souche de lactobacilles vivants résistants à la nisine pour réduire une post-acidification dans un produit alimentaire.

2. Utilisation selon la revendication 1, dans laquelle les lactobacilles sont sélectionnés dans le groupe constitué de *Lactobacillus helveticus, L. delbrueckii subsp. bulgaricus, L. delbrueckii subsp. delbrueckii, L. delbrueckii subsp. lactis, L. casei, L. paracasei, L. acidophilus, L. rhamnosus, L. plantarum, L. reuteri, L. brevis* et *L. fermentum.*

3. Utilisation selon la revendication 2, dans laquelle les lactobacilles sont en particulier choisis parmi *L. helveticus, L. delbrueckii* et leurs sous-espèces, en particulier *L. delbrueckii subsp. bulgaricus.*

4. Utilisation selon la revendication 3, dans laquelle les lactobacilles sont choisis dans le groupe comprenant CNCM I-4452, CNCM I-4453 et CNCM I-4454.

5. Utilisation selon les revendications 1 à 4, dans laquelle la souche a une résistance à la nisine de concentration minimale inhibitrice d'au moins 6,25 µg/ml, en particulier de concentration minimale inhibitrice d'au moins 12,5 µg/ml.

6. Utilisation selon les revendications 1 à 5, dans laquelle le produit alimentaire est choisi dans le groupe comprenant des produits laitiers, du lait, des jus de fruits, des produits à base de légumes, des préparations pour nourrissons, du lait en poudre.

7. Utilisation selon les revendications 1 à 6, dans laquelle les produits fermentés sont choisis dans le groupe comprenant des produits laitiers fermentés, des jus fermentés, des produits végétaux fermentés.

8. Procédé de réduction d'une post-acidification dans des produits alimentaires dans lequel ledit procédé comprenant une étape consistant à ajouter au moins une souche de lactobacilles vivants résistants à la nisine dans une matrice alimentaire.

9. Procédé selon la revendication 8, dans lequel les lactobacilles sont sélectionnés dans le groupe constitué de *Lactobacillus helveticus, L. bulgaricus, L. casei, L. paracasei, L. acidophilus, L. rhamnosus, L. plantarum, L. reuteri, L. delbrueckii subsp. bulgaricus* et *L. fermentum.*

10. Procédé selon la revendication 9, dans lequel les lactobacilles sont sélectionnés dans le groupe comprenant CNCM I-4452, CNCM I-4453 et CNCM I-4454.

11. Procédé selon les revendications 8 à 10, dans lequel la souche a une résistance à la nisine de concentration minimale inhibitrice d'au moins 6,25 µg/ml, en particulier de concentration minimale inhibitrice d'au moins 12,5 µg/ml.

12. Procédé selon les revendications 8 à 11, dans lequel l'addition des lactobacilles résistants à la nisine est effectuée pendant, avant ou après l'étape de fermentation du produit alimentaire.

13. Procédé selon les revendications 8 à 12, dans lequel la fermentation est effectuée en présence d'au moins une autre bactérie vivante.

14. Procédé selon les revendications 8 à 13, dans lequel ladite autre bactérie vivante est une bactérie lactique vivante, choisie dans le groupe constitué de *Streptococcus spp. Lactobacillus spp,* en particulier *L. bulgaricus, L. acidophilus* et *L. casei; L. helveticus, Lactococcus spp.* et *Bifidobacterium spp..*

15. Procédé selon l'une quelconque des revendications précédentes dans lequel ledit procédé comprend les étapes consistant à :
a) fournir une souche de lactobacilles vivants résistants à la nisine,
b) inoculer une matrice alimentaire avec la souche obtenue à l'étape a),
c) fermenter le milieu inoculé à une température de 4°C à 50°C, jusqu'à atteindre un pH souhaité
d) récupérer et stocker le produit obtenu à l'étape c), à une température de 4°C à 40°C pendant au moins 28 jours.

16. Souche mutante de *L. bulgaricus,* déposée le 10 mars 2011 auprès de la CNCM sous le numéro I-4452.

17. Souche mutante de *L. bulgaricus,* déposée le 10 mars 2011 auprès de la CNCM sous le numéro I-4453.

18. Souche mutante de *L. helveticus,* déposée le 10 mars 2011 auprès de la CNCM sous le numéro I-4454.
